# EUROPEAN PATENT SPECIFICATION

(11) **EP 0 540 314 B1**
(45) Date of publication and mention of the grant of the patent: **10.07.1996**
(21) Application number: 92309880.0
(22) Date of filing: 28.10.1992
(51) Int. Cl.: C12P 21/08, G01N 33/577

(54) **Monoclonal antibodies to polyacrylate polymers**
Monoklonale Antikörper gegen Polyacrylat-Polymeren
Anticorps monoclonaux contre des polymères de polyacrylate

(30) Priority: 31.10.1991 US 786154
(43) Date of publication of application: 05.05.1993
(73) Proprietor: NALCO CHEMICAL COMPANY, Naperville Illinois 60563-1198 (US)
(72) Inventor: Wetegrove, Robert L., Winfield, IL 60190 (US); Bruehl, Richard E., Oakland, CA 94606 (US); Balakrishnan, Krishna, Richmond, CA 94805 (US)
(74) Representative: Harrison, David Christopher

(56) References cited:
- EP-A- 0 535 347

## Description

The invention relates generally to monoclonal antibodies directed toward polyacrylate and to antibody assays for the detection of polyacrylate. A method for the detection and monitoring of polyacrylate in commercial water systems is used to optimize treatment of the water to prevent mineral deposits.

Preservation of containers used for holding water for commercial purposes requires removal of mineral deposits.

For example, as described in US-A- 4,756,881 and 4,752,443, water-soluble sulfonated copolymers of acrylic acid and acrylamide (hereinafter referred to as "sulfonated copolymers") are used in the treatment of industrial cooling water to prevent corrosion and mineral deposits (scale). Generally, the active sulfonated copolymers remove dissolved minerals from the cooling water by complexing with the mineral. over time, the complexation sites of the sulfonated copolymer molecules become saturated and the copolymer molecules become inactive, unable to remove any additional minerals from the cooling water.

To prevent corrosion and scale damage to machinery, as the polymers are inactivated they must be removed and replaced by active sulfonated copolymer. Thus, active sulfonated copolymer must be continually fed into the cooling water to replace the inactive sulfonated copolymer. Maintaining the proper feed level for the active sulfonated copolymer is essential for optimum performance of the cooling water system. An improper feed rate can lead to serious problems. For example, insufficient active sulfonated copolymer can result in the water treatment being overwhelmed by dissolved minerals, thereby causing severe corrosion or scale deposit. On the other hand, maintaining too high a level of the active polymer is very expensive and is an inefficient method for treating industrial cooling water.

Although several methods are available for determining the total concentration of sulfonated copolymer in an industrial cooling water system, i.e., active plus inactive sulfonated copolymer, these techniques are unsatisfactory since they only determine the concentration of total sulfonated copolymer, and do not measure the concentration of the active sulfonated copolymer. Moreover, these methods suffer from lack of specificity or poor sensitivity. For example, the older methods for detecting sulfonated copolymers include colloid titration with PVSK complexation with hyamine 1622, or reaction of excess magnesium with chrome azurol S. The above tests detect any polyanionic material and have a detection threshold of about 50 ppm polymer. Presently, the total amount of active sulfonated polymer in an industrial cooling water system cannot be inexpensively and rapidly determined. Therefore, monoclonal antibodies were developed which are directed toward the sulfonated copolymers in order to detect them and to monitor their concentration to optimize the effectiveness of treatment.

Another composition, water-soluble acrylate homopolymers (hereinafter referred to as "polyacrylate") are used in the treatment of industrial boiler water to prevent corrosion and mineral deposits (scale). Generally, the polyacrylate removes dissolved minerals from the boiler water by complexing with the mineral. Over time, the complexation sites of the polyacrylate become saturated and the molecules become inactive, unable to remove any additional minerals from the boiler water.

To prevent corrosion and scale damage to machinery, as the polyacrylate polymers are inactivated they must be removed and replaced by active polyacrylate. Thus, active polyacrylate must be continually fed into the boiler water. Maintaining the proper feed level of polyacrylate is essential for optimum performance of the boiler water system. An improper feed rate can lead to serious problems. For example, insufficient levels can result in the water treatment being overwhelmed by dissolved minerals, thereby causing severe corrosion or scale deposit. On the other hand, maintaining too high a level is very expensive and is an inefficient method for treating industrial boiler water.

Although several methods are available for determining the concentration of polyacrylate in boiler water, these methods suffer from lack of specificity or poor sensitivity. For example, the older methods for detecting polyacrylates include colloid titration with PVSK, complexation with hyamine 1622, or reaction of excess magnesium with chrome azurol S. The above tests detect any polyanionic material and have a detection threshold of about 50 ppm polymer. Presently, the amount of polyacrylate in an industrial boiler water system cannot be inexpensively and rapidly determined. Therefore, monoclonal antibodies were developed which are directed toward polyacrylate in order to detect the polyacrylate and to monitor its concentrated to optimize treatment.

Our EP-A-0535 347, published 7 April 1993 and therefore falling within the terms of Article 54(3)EPC, discloses such antibodies and their use in relation to polyacrylate copolymers. In contrast, one aspect of the present invention is directed to the use of a monoclonal antibody for detection or assay of polyacrylate homopolymer. When used in some detection formats, the present monoclonal antibodies have an absolute specificity and a lower limit of detection in the femtogram per ml range.

The monoclonal antibodies are produced by hybridoma cell lines. one preferred hybridoma cell line of the invention is hybridoma cell line 6E2-H1-G4. Another preferred hybridoma cell line of the invention is hybridoma cell line 4F12-C12. A still further preferred hybridoma cell line is 4B1-H6-E10.

Another aspect of the invention is directed to a method of manufacturing a monoclonal antibody having an affinity to polyacrylate homopolymer. The inventive method including the steps of: a) immunizing a mammal with polyacrylate homopolymer attached to a carrier protein; b) preparing a hybridoma cell producing the monoclonal antibody from cells removed from the immunized mammal; c) cloning the hybridoma cell to produce a hybridoma cell line; and d) extracting the monoclonal antibody from the hybridoma cell line.

When sulfonated copolymers of acrylic acid and acrylamide were used for immunizing, various hybridomas can be obtained, in accordance with the present invention. These are a source of monoclonal antibodies, which complex with polyacrylate and are suitable for use in its detection. Using the methods disclosed herein, multiple hybridomas were produced which were suitable for use in the present invention. Therefore, it is to be expected that others may be readily produced using the disclosed methods. However, resultig antibodies vary in binding profiles and specificity. For example, a preferred hybridoma cell line for this purpose is hybridoma cell line 6E2-H1-G4. Accordingly, in other preferred embodiments of the invention, the hybridoma cell line is hybridoma cell line 4F12-C12 or 4B1-H6-E10.

A further aspect of the invention is directed to a process for the determination of the presence or concentration of polyacrylate homopolymer in a fluid. The inventive process including the step of incubating a sample of the fluid containing the polyacrylate homopolymer with a monoclonal antibody having an affinity for the polyacrylate homopolymer, the monoclonal antibody being bound to a solid carrier. According to a preferred embodiment, the fluid is boiler water.

### Brief Description of the Drawings

FIG. 1 is a graphic illustration of the binding profile of the monoclonal antibodies from the hybridoma cell lines 4B1-H6-E10, 4F12-C12, 6E2-H1-G4, 4D4-C9-F6 and 6D12-H9-H3 to polyacrylate (35K), polyacrylate conjugated to bovine serum albumin (35K-BSA) and bovine serum albumin (BSA);
FIG. 2 graphically illustrates the binding profile of the monoclonal antibody from the hybridoma cell line 6E2-H1-G4 to 35K-BSA 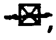 35K 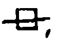 and BSA 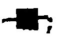 and
FIG. 3 is a graphic illustration of the binding profile of the monoclonal antibody from hybridoma cell line 4F12-C12 to 35K-BSA 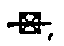 35K 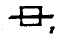 and BSA 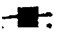

### Description of the Preferred Embodiments

Hybridoma cell lines producing monoclonal antibodies having an affinity for polyacrylate homopolymers have been invented and are herein described and claimed. The monoclonal antibodies of the invention bind to polyacrylate with varying degrees of specificity. By using two different assay formats, it has been determined that these monoclonal antibodies can recognize polyacrylate at concentrations ranging from zero to a few hundred parts per million. Examples described herein demonstrate the feasibility of using the monoclonal antibodies of the invention to determine the presence or concentration of polyacrylate in fluids, such as water samples from industrial boilers.

According to one embodiment of the invention, a monoclonal antibody having an affinity to polyacrylate is used in an Enzyme Linked Immunosorbent Assay (ELISA) for determining the concentration of polyacrylate in a fluid sample. ELISA formats using the monoclonal antibodies of the invention are preferred for measuring the concentration of polyacrylate in fluid samples. Three preferred immunoassay strategies are sandwich ELISA, competition ELISA and indirect ELISA. Although several antibodies described herein are useful in assaying polyacrylate in a fluid sample, the two most preferred monoclonal antibodies are those produced by hybridoma cell lines 6E2-H1-G4 (deposited as ATCC accession number HB 11015) and 4F12-C12 because these two clones have shown the greatest anti-polyacrylate specificity in indirect ELISAS.

According to one preferred embodiment of the invention, a sandwich ELISA will be used for measuring the concentration of polyacrylate. Preferably, purified anti-polyacrylate antibody will be used as a capture antibody. The antibody will be adsorbed to wells of 96-well microtiter plates. Test samples containing polyacrylate will be added and allowed to bind to the capture antibody. In the next step, enzyme-labelled anti-polyacrylate antibody will be added and allowed to bind to the exposed antigenic sites on the polyacrylate molecules captured by the coating antibody. The amount of enzyme labelled antibody retained after washing would therefore correlate directly with the amount of polyacrylate in the sample. The level of enzyme label bound in each well will be quantitated by incubation with a chromogenic enzyme substrate and measurement of the resulting color change. A standard curve will be generated using known concentrations of polyacrylate polymer. This approach has the advantage of higher sensitivity normally associated with sandwich ELISAs.

In another preferred embodiment, a competition ELISA will be used to measure the polyacrylate for a fluid sample. According to this embodiment, purified antipolyacrylate antibody will be adsorbed onto wells of 96-well microtiter plates. Test samples containing polyacrylate will be added to the plates along with known quantities of enzyme-labelled polyacrylate polymer. The amount of enzyme-labelled polyacrylate retained after washing will be inversely proportional to the amount of polyacrylate originally present in the test samples. The amount of enzyme present will be measured by the addition of a suitable chromogenic substrate. Standard curves will be generated using known quantities of polyacrylate. This particular format has the advantage of being essentially a one-step assay with only one incubation period.

According to a still further embodiment of the invention, indirect ELISA assay will be utilized to measure the concentration of polyacrylate in a fluid. According to the embodiment, known quantities of Bovine Serum Albumin (BSA-polyacrylate) conjugate will be adsorbed to replicate wells of 96 well microtiter plates. Next, various dilutions of the anti-polyacrylate monoclonal antibody will be allowed to bind to the immobilized polyacrylate. Enzyme-labelled goat anti-mouse Ig will be added next and allowed to bind to the primary antibody. A chromogenic substrate will then be added which will be converted to a colored product by the bound enzyme. The resultant color change will be quantified by measuring the absorbance (optical density) at 492 mm. The amount of color change will be proportional to the amount of enzyme-labelled antibody retained and thus will correlate directly with the amount of anti-polyacrylate antibody which was able to initially bind to the immobilized polyacrylate. The results of this experiment will yield the optimal concentration of anti-polyacrylate antibody to use in the assay.

With the appropriate concentration of antibody defined for an indirect ELISA, inhibition of binding between the monoclonal antibody and the polyacrylate-coated microtiter plates by polyacrylate present in solution will be determined. The monoclonal anti-polyacrylate primary antibody will be incubated with various concentrations of polyacrylate before being added to the polyacrylate-coated microtiter plates. A standard curve will then be prepared by plotting the percentage inhibition as a function of free polyacrylate concentration. This will be carried out for each fixed quantity of immobilized polyacrylate and the primary antibody. This assay format will be suitable if this standard curve shows a steep dose-response at the concentrations that are relevant to those which occur in cooling water samples. It has the advantage of not requiring any additional purification or modification of the anti-polyacrylate monoclonal antibody.

Any of the assays discussed above could be further developed and refined using other solid support systems such as coated tubes and polymer membranes. However, the sandwich ELISA or the competition ELISA formats are preferred since they could be designed as a "dipstick" assay. Nevertheless, any of the above assay formats are intended to be used with the monoclonal antibodies of the present invention to measure the concentration of polyacrylate in a fluid sample.

The following examples are presented to describe preferred embodiments and utilities of the invention and are not meant to limit the invention unless otherwise stated in the claims appended hereto.

### EXAMPLE 1. Antigen synthesis: Polyacrylate

The antigens were prepared as follows:

Ten mg of the polyacrylate (35K) having a molecular weight of about 35KD was obtained from the Nalco Chemical Company. The polyacrylate was first derivatized with the coupling agent, 1-ethyl 3(3'-dimethylamino-propyl) carbodiimide (EDC) at a pH of 5.0. After a 15 minute incubation at room temperature, 10 mg of Bovine Serum Albumin (BSA) was added and allowed to react at pH 7.0 for 4 hours, also at room temperature. Small molecular weight side products were removed by overnight dialysis at 4°C. Gel electrophoresis of the BSA conjugate indicated successful conjugation with approximately one to four polymer molecules conjugated per molecule of BSA.

### EXAMPLE 2. Utility of Polyacrylate-BSA Conjugate in Indirect ELISA Screening.

The BSA conjugates of the polyacrylate were tested for adsorption onto microtiter plates by performing an indirect ELISA using rabbit anti-BSA antiserum. Plates were coated with the conjugates at a concentration of 0.01 mg/ml (0.5 µg/well) in phosphate-buffered saline, pH 7.2 (PBS). Unoccupied sites on the plates were blocked with a 5% solution of non-fat milk in PBS. Dilutions of rabbit anti-BSA antiserum were incubated with the plate. Horseradish peroxidase (HRP) labelled goat anti-rabbit antibody was then allowed to bind to the primary anti-BSA antibody. The bound enzyme (HRP) was then quantitated with a chromogenic substrate. These assays were performed in duplicate. The results of the assay are summarized in Table 1. These results clearly indicate that the BSA-polyacrylate conjugates bound strongly to the ELISA plates, thus confirming the utility of these conjugates for the screening of antipolyacrylate antibodies in an indirect ELISA format.

**TABLE 1**

| Indirect ELISA with Rb anti-BSA sera | | | |
|---|---|---|---|
| Set | Antigen | OD_{1/2max} | Titer value |
| | | | |
| #1 | 35K-BSA | 0.94 | 1:7100 |
| | BSA | 0.87 | 1:14,000 |
| | No Ag | 0.31 | 1:50 |
| | | | |
| #2 | 35K-BSA | 0.92 | 1:7700 |
| | BSA | 0.86 | 1:13,000 |
| | No Ag | 0.36 | < 1:50 |

results; antibody titer being defined as the dilution of the sample sera which yields an optical density that is one-half of the maximum signal obtained (OD_{1/2max}) in an indirect ELISA. Table 1 presents the titer results obtained for all four groups of animals.

### EXAMPLE 3. Identification of Useful Hybridomas

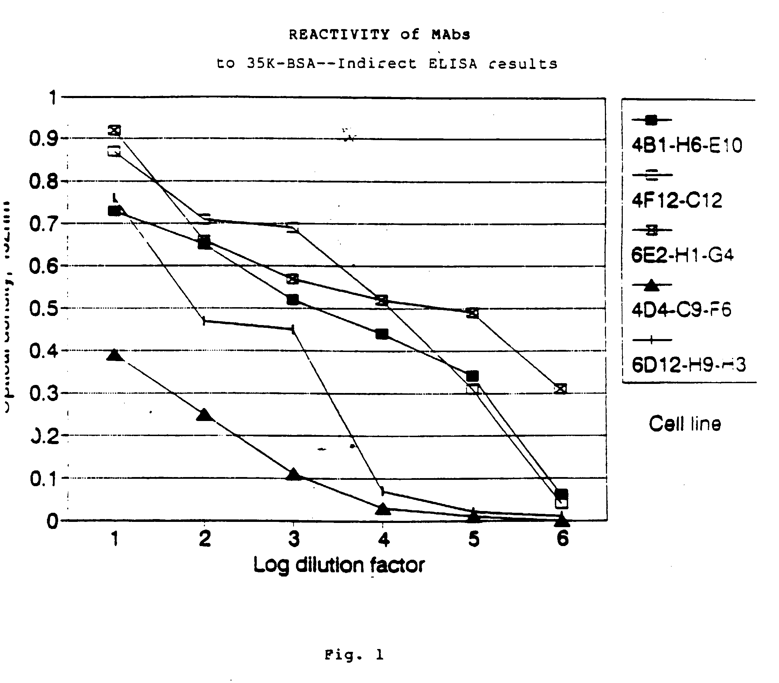

Monoclonal antibodies produced by five cell lines were assayed by indirect ELISA for binding to polyacrylate-BSA conjugates. The five cell lines tested were:
481-H6-E10, 4F12-C12, 6E2-H1-G4, 4D4-C9-F6 and 6D12-H9-H3. These assays were performed in duplicate and the results of the assays are summarized in Figure 1.

### EXAMPLE 4. Antibody scale-up

Hybridoma cell lines 6E2-H1-G4 and 4H2-C12 were scaled up by growing them in vitro for several passages and injecting them into a group of pristane-primed Balb/c mice. The cells grew as ascites tumors in these mice and the resultant fluid accumulated in their peritoneal cavities was harvested. The ascites fluid was enriched in the desired anti-polyacrylate antibody which was purified and used in the assays described below.

### EXAMPLE 5. Antibody Purification

Depending on the assay format being pursued, antibodies will be required either as unpurified ascites or as a highly purified immunoglobulin preparation. Antibodies were purified from ascites fluid by a variety of methods, depending on the antibody isotype and the sensitivity of the antibody to various buffere. The clones 6E2-H1-G4 and 4F12-C12 identified in Example 3 are of the IgG isotype, and accordingly, they were purified by using the pseudo-affinity matrix Protein A.

### EXAMPLE 6. Reactivity Profile of the Anti-polyacrylate Monoclonal Antibodies

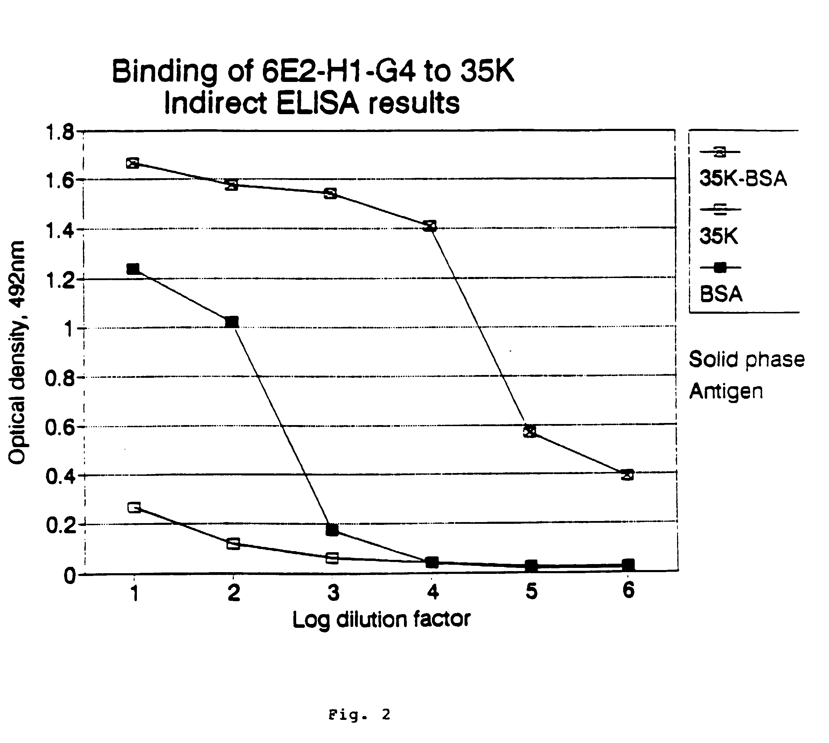

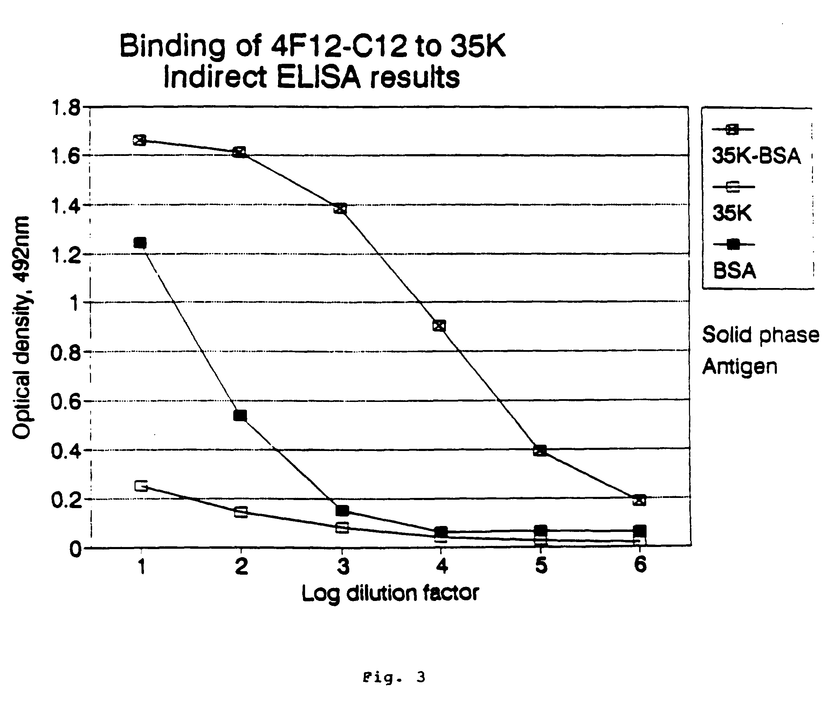

The binding specificity of the different anti-polyacrylate monoclonal antibodies was tested by an indirect ELISA. Replicate wells were coated with 500 ng per well of the following panel of antigens: BSA-polyacrylate, polyacrylate, or BSA. Serial dilutions of the ascites fluid ranging from 1:10 to 1:10⁶ in steps of 10 were incubated with the various antigen coated plates. After an incubation with the ascites fluid samples, the plates were washed and incubated with HRP-labelled goat anti-mouse Ig. Finally, the quantity of enzyme (HRP) retained in each well was quantified using H₂O₂ and a chromogenic substrate. The optical density readout at 492 mm was recorded using an automated ELISA reader and the results are summarized in Figs. 2 and 3.

Of the two different monoclonal antibodies whose results are shown in Figs. 2 and 3, cell lines 6E2-H1-G4 and 4F12-C12 show a dramatically higher binding specificity for BSA-polyacrylate as compared to BSA or polyacrylate alone. These results confirm the earlier findings shown in Fig. 1, where these studies were carried out with tissue culture supernatants from these cell lines.

Changes can be made in the composition, operation and arrangement of the various cell lines, monoclonal antibodies, steps and procedures described herein without departing from the concept and scope of the invention.

Mouse hybridoma cells 6E2-H1-G4 and 6D12-H9-G3 have been deposited at the American Type Culture Collection on 9 April 1992 and have been designated HB 11015 and HB 11016 respectively.

## Claims

1. The use of a monoclonal antibody produced by hybridoma cell line 6E2-H1-G4 (ATCC HB 11015) for detection or assay of polyacrylate homopolymer.

2. A method of producing a monoclonal antibody having an affinity to polyacrylate homopolymer, the method including the steps of:
(a) immunizing a mammal with the polyacrylate homopolymer conjugated with a carrier protein;
(b) preparing a hybridoma cell producing the monoclonal antibody by fusing splenocytes from the immunized mammal with plasmocytoma cells;
(c) establishing said hybridoma cell to produce a cell line; and
(d) isolating said monoclonal antibody from said hybridoma cell line.

3. A method according to claim 2 wherein the mammal is a mouse and the hybridoma cell line is hybridoma cell line 6E2-H1-G4 (ATCC HB 11015).

4. A process for the determination of the presence or concentration of a polyacrylate homopolymer in a fluid, the process including the step of incubating a sample of the fluid containing the polyacrylate homopolymer with a monoclonal antibody having an affinity for the polyacrylate homopolymer, the monoclonal antibody being bound to a solid carrier, and detecting and measuring the degree of the binding of monoclonal antibody, from which the presence of polyacrylate homopolymer can be inferred and concentration of said polyacrylate homopolymer can be determined.

5. A process according to claim 4 wherein the antigen-antibody binding is detected and measured by an enzyme-linked immunosorbent assay.

## Patentansprüche

1. Verwendung eines von der Hybridoma-Zellinie 6E2-H1-G4 (ATCC HB 11015) hergestellten monoklonalen Antikörpers zur Detektion oder zum Assay von PolyacrylatHomopolymer.

2. Verfahren zur Herstellung eines monoklonalen Antikörpers mit einer Affinität für Polyacrylat-Homopolymer, wobei das Verfahren folgende Schritte umfaßt:
(a) das Immunisieren eines Säugetiers mit dem mit einem Trägerprotein konjugierten Polyacrylat-Homopolymer;
(b) das Herstellen einer Hybridomazelle, die den monoklonalen Antikörper erzeugt, durch Verschmelzen von Splenozyten vom immunisierten Säugetier mit Plasmozytomazellen;
(c) das Etablieren der Hybridomazelle, um eine Zellinie zu erzeugen; und
(d) das Isolieren des monoklonalen Antikörpers aus der Hybridoma-Zellinie.

3. Verfahren nach Anspruch 2, worin das Säugetier eine Maus ist und die Hybridoma-Zellinie die Hybridoma-Zellinie 6E2-H1-G4 (ATCC HB 11015) ist.

4. Verfahren zur Ermittlung des Vorhandenseins oder der Konzentration eines polyacrylat-Homopolymers in einem Fluid, wobei das Verfahren den Schritt des Inkubierens einer Probe des das Polyacrylat-Homopolymer enthaltenden Fluids mit einem monoklonalen Antikörper mit einer Affinität für das Polyacrylat-Homopolymer, wobei der monoklonale Antikörper an einen festen Träger gebunden ist, sowie das Detektieren und Messen des Bindungsgrades des monoklonalen Antikörpers umfaßt, aus dem auf das Vorhandensein von Polyacrylat-Homopolymer geschlossen und die Konzentration des Polyacrylat-Homopolymers ermittelt werden kann.

5. Verfahren nach Anspruch 4, worin die Antigen-Antikörper-Bindung durch ein enzymgebundenes Immunosorbens-Assay detektiert und gemessen wird.

## Revendications

1. Utilisation d'un anticorps monoclonal produit par la lignée de cellules d'hybridome 6E2-H1-G4 (ATCC HB 11015) pour la détection ou l'analyse d'un homopolymère de polyacrylate.

2. Méthode de production d'un anticorps monoclonal ayant une affinité pour un homopolymère de polyacrylate, la méthode comprenant les étapes de :
(a) immuniser un mammifère par l'homopolymère de polyacrylate conjugué à une protéine porteuse ;
(b) préparer une cellule d'hybridome produisant l'anticorps monoclonal par fusion de splénocytes du mammifère immunisé à des cellules de plasmocytome ;
(c) établir ladite cellule d'hybridome pour produire une lignée de cellules ; et
(d) isoler ledit anticorps monoclonal de ladite lignée de cellules d'hybridome.

3. Méthode selon la revendication 2 où le mammifère est une souris et la lignée de cellules d'hybridome est une lignée de cellules d'hybridome 6E2-H1-G4 (ATCC HB 11015).

4. Procédé pour la détermination de la présence ou de la concentration d'un homopolymère de polyacrylate dans un fluide, le procédé comprenant l'étape de soumettre un échantillon du fluide contenant l'homopolymère de polyacrylate à incubation avec un anticorps monoclonal ayant une affinité pour l'homopolymère de polyacrylate, l'anticorps monoclonal étant lié à un support solide, et de détecter et de mesurer le degré de liaison de l'anticorps monoclonal, d'où on peut déduire la présence de l'homopolymère de polyacrylate et la concentration dudit homopolymère de polyacrylate peut être déterminée.

5. Procédé selon la revendication 4 où la liaison antigène-anticorps est détectée et mesurée par une analyse d'immunosorbant lié à l'enzyme.
